# EUROPEAN PATENT APPLICATION

(11) **EP 1 557 660 A1**
(43) Date of publication of application: **27.07.2005**
(21) Application number: 03758905.8
(22) Date of filing: 24.10.2003
(51) Int. Cl.: G01N 21/78, G01N 33/52, G01N 31/22

(54) **METHOD OF CORRECTION AT SAMPLE ANALYSIS, ANALYZER AND ANALYTICAL EQUIPMENT**

(30) Priority: 28.10.2002 JP 2002312960
(71) Applicant: ARKRAY, Inc., Kyoto-shi, Kyoto 601-8045 (JP)
(72) Inventor: NAKANO, Hajime c/o ARKRAY, Inc., Kyoto-shi, Kyoto 601-8045 (JP); TAGUCHI, Takayuki c/o ARKRAY, Inc., Kyoto-shi, Kyoto 601-8045 (JP)
(74) Representative: Pluckrose, Anthony William
(86) International application number: PCT/JP2003/013669
(87) International publication number: WO 2004/038392

(57) **Abstract**

The present invention relates to a technique for correcting during analysis of a sample liquid for the effects of reaction system characteristics on analytical results when a sample is analyzed using a reaction system from the reaction of a sample and a reagent. In the present invention, a method is provided of applying correction when analyzing each item in a method of analyzing a plurality of analysis items based on a reaction liquid from the reaction of a sample and a reagent. In this method, correction is performed based on the same blank measurement results with respect to those of a plurality of types of analysis items which have similar reaction conditions during analysis.

## Description

### TECHNICAL FIELD

The present invention relates to a technique for correcting during analysis of a sample liquid for the effect of reaction system characteristics on analysis results when a sample is analyzed using a reaction system from reaction of a sample and a reagent.

### BACKGROUND ART

Methods of analyzing a sample include those using optical means. For example, there are methods in which a reaction system is constructed by reacting a sample and a reagent in an analyzing instrument, the reaction system is exposed to light in an analyzing apparatus and the sample is analyzed based on the response (such as amount of transmitted light or reflected light) from the reaction system (see for example U.S. Patent No. 3526480, Specifications). When such an analysis method is adopted, the analytical accuracy is known to be affected by pigment components (such a bilirubin (Bil) and hemoglobin (Hb)) or lipids contained in the sample.

For example, if a sample contains pigment components more light will be absorbed in the reaction system. In addition, the amount of Bil and Hb contained in a sample is not always the same from sample to sample, and pigment components such as Bil and Hb oxidize over time when a sample is stored, altering the absorption spectrum. Consequently, the amount of light absorbed in a reaction system differs depending on the amount of pigment components and the degree to which the pigment components have oxidized. Moreover, the degree to which Bil and Hb are oxidized is affected by pH, and Bil and Hb tend to oxidize more easily in alkaline environments. Consequently, because the degree to which Bil and Hb oxidize differs depending on the pH when the sample and reagent are reacted, the amount of light absorbed in the reaction system differs depending on the reaction conditions in the reaction system.

When a reagent containing a surfactant is used, because lipids are dissolved in the reaction system by nonionic surfactants during analysis, the degree of cloudiness (milkiness) is different before and after reaction. Consequently, in systems containing surfactants the effect on response is different before and after the reaction.

Blank correction is normally used to correct for this decrease in analytical accuracy. Blank correction is performed by obtaining correction data using a measurement system comprising a sample and a blank reagent, and correcting the analysis results based on this correction data. For example, when considering the effect of pigment components blank measurement must be performed in a system which comprises the sample and has the same pH as the reaction system. When considering the effect of lipids, on the other hand, blank measurement must be performed in a system which comprises the sample and a surfactant of the same type and quantity as in the reaction system.

On the other hand, when testing urine for example multiple tests are often performed on a single sample, and the kinds of factors which affect analytical accuracy and the degree to which they do so are different for each analysis item. Consequently, when analyzing multiple items from a single sample blank correction is preferably performed for each analysis item.

However, in methods in which as many blank measurements are performed as there are analysis items, the problems explained below occur because so many blank measurements are needed. That is, first there is the problem of cost because the total amount of reagent needed for blank correction is so large. Second, because sample is required for each blank measurement the total amount of sample required for analysis is large, increasing the burden on the test subject when analyzing biological samples such as urine and blood. Third, because more reaction regions need to be reserved for blank measurement in the analyzing instrument in order to perform multiple analyses in one analyzing instrument, the analyzing instrument becomes larger or the space assigned to each reaction region becomes smaller.

### DISCLOSURE OF THE INVENTION

It is an object of the present invention to allow cost-effective blank correction to be performed based on small samples without affecting the small size of the analyzing instrument

In a method of performing analysis with respect to a plurality of types of analysis items on the basis of a reaction liquid from reaction of a sample and a reagent, the first aspect of the present invention provides a method of correction during sample analysis which is a method of applying correction when analyzing the aforementioned analysis items, wherein correction is applied based on the same blank measurement results with respect to those plurality of analysis items out of the aforementioned plurality of analysis items for which the reaction conditions during analysis are similar.

In a preferred embodiment, the plurality of analysis items are separated into a plurality of groups with the members of each group sharing similar reaction conditions during analysis, a plurality of blank measurements each with differing measurement conditions corresponding to the plurality of groups, a plurality of blank measurements with measurement conditions different from one another are performed corresponding to the plurality of groups, and correction is applied based on the blank measurement results corresponding for each of the groups in analyzing the individual analysis items that make up the group.

In the second aspect of the present invention, an analyzer is provided which is an analyzer for analyzing a plurality of specific components in a sample on the basis of a reaction liquid from reaction of a sample and a reagent, wherein in an analyzer equipped with a computation means for performing computations necessary for analyzing a plurality of specific components in a sample, the aforementioned computation means is constructed so as to apply correction based on correction data obtained based on the same blank measurement results for a plurality of specific components having similar reaction conditions during analysis when performing computations for analyzing the aforementioned plurality of specific components.

This analyzer preferably also comprises a correction means for obtaining the aforementioned correction data based on the blank measurement results.

In the third aspect of the present invention, an analyzing instrument is provided which is an analyzing instrument equipped with a plurality of analysis reagent parts each comprising a different reagent and one or multiple blank measurement reagent parts, wherein the aforementioned one or multiple blank measurement reagent parts are shared by those of the aforementioned plurality of analysis reagent parts which have similar reaction conditions during analysis.

In the present invention, correction can also be performed based on the results of two or more blank measurements for some of the plurality of analysis items. Such correction can be performed in the computation part of the analyzer.

The reaction conditions which are the criteria for grouping blank measurements include the attributes and composition of the reaction liquid for example. A typical attribute of a reaction liquid is the pH of the reaction liquid. Typically, composition of the reaction liquid includes whether or not it contains a surfactant. Consequently, in the present invention analysis items having similar reaction conditions are for example those for which the pH of the reaction liquid is similar during analysis, or those for which a surfactant is included in the reaction liquid during analysis. Of course, blank measurements can also be grouped for a plurality of analysis items based on reaction conditions other than pH and the presence or absence of a surfactant.

Blank measurement in the present invention is performed for example in at least one measurement system selected from an acidic blank measurement system, a neutral blank measurement system, an alkaline blank measurement system and a surfactant blank measurement system comprising a surfactant.

The acidic blank measurement system is set to a value selected in the range of pH 3.0 to 5.5 for example, and is constructed from a citric acid buffer liquid or other carboxylic acid type buffer liquid. In this acidic blank measurement system, the salt content of the buffer is 0.05 to 0.5 mol/L for example.

The neutral blank measurement system is set to a pH 7.0 near for example, and is constructed from a phosphoric acid buffer. In this neutral blank measurement system, the salt concentration of the buffer is 0.05 to 0.5 mol/L for example.

The alkaline blank measurement system is set to a value selected in the range of pH 8.5 to 11.0 for example, and is constructed from a (cyclohexylamino)ethanesulfonic acid (CHES) buffer or (cyclohexylamino)propanesulfonic acid (CAPS) buffer. In this alkaline blank measurement system, the salt concentration of the buffer is 0.05 to 0.5 mol/L for example.

The acidic blank measurement system, neutral blank measurement system and alkaline blank measurement system may also be constructed from buffers containing a plurality of kinds of salts. For example, the alkaline blank measurement system can be constructed from Good's buffer.

The surfactant blank measurement system is constructed to contain a buffer and a nonionic surfactant for example so as to be capable of solubilizing triglyceride neutral fat and other lipids. The concentration of surfactant in this surfactant blank measurement system is 0.01 to 1.0 wt% for example. The surfactant blank measurement system is preferably adjusted to an acidic system (pH 3.0 to 5.5), a neutral system (near pH 7.0) or an alkaline system (pH 8.5 to 11.0) according to the type of buffer used. The buffer used in this case is selected according to the desired pH, and buffers similar to those given as examples above when explaining the acidic blank measurement system, neutral blank measurement system and alkaline blank measurement system can be used for example.

For example, for items such as albumin which are analyzed in an acidic system containing a surfactant, the surfactant blank measurement system is constructed as an acidic system. However, the surfactant blank measurement system can also be constructed as a neutral system and blank correction performed based on the results of both this blank measurement system and an acidic blank measurement system. Of course, for items which are analyzed in an alkaline reaction system containing a surfactant, blank correction can also be performed based on the results of both a neutral surfactant blank measurement system and an alkaline blank measurement system.

Examples of nonionic surfactants which can be used in the present invention include ether, ether ester, ester and nitrogen-containing surfactants.

Examples of ether-type surfactants include polyoxyethylene alkyl ethers, polyoxyethylene secondary alcohol ethers, polyoxyethylene alkylphenyl ethers, polyoxyethylene sterol ethers, polyoxyethylene lanolin derivatives, ethylene oxide derivatives of condensed alkyl phenol formalin, polyoxyethylene polyoxypropylene block polymers and polyoxyethylene polyoxypropylene alkyl ethers.

Examples of ether ester-type surfactants include polyoxyethylene glycerin fatty acid esters, polyoxyethylene castor oil, polyoxyethylene hardened castor oil, polyoxyethylene sorbitan fatty acid esters and polyoxyethylene sorbitol fatty acid esters.

Examples of ester-type surfactants include polyethylene glycol fatty acid esters, fatty acid monoglycerides, polyglycerin fatty acid esters, sorbitan fatty acid esters, propylene glycol fatty acid esters and sucrose fatty acid esters.

Examples of nitrogen-containing surfactants include fatty acid alkanolamides, polyoxyethylene fatty acid amides, polyoxyethylene alkylamines, alkylamine oxides and the like.

Typical samples include urine and blood. Examples of analysis items include albumin (Alb), total bilirubin (T-Bil), inorganic phosphorus (IP), glucose (Glu), uric acid (UA), urea nitrogen (BUN), aspartate aminotransferase (GOT), alanine aminotransferase (GPT), creatine phosphokinase (CPK), amylase (Amy), gammaglutamyl transpeptidase (GGT), creatinine (Cre), total protein (TP), calcium (Ca), lactic dehydrogenase (LDH), alkaline phosphatase (ALP), magnesium (Mg), fructosamine (FRA), total cholesterol (T-Cho), high density cholesterol (HDL-Cho) and triglyceride neutral fat (TG).

Of the listed analysis items, for example Alb, T-Bil or IP is corrected based on the results of blank measurement performed in an acidic blank measurement system, Glu, UA, BUN, GOT, GPT, CPK, Amy, CGT or Cre is corrected based on the results of blank measurement performed in a neutral blank measurement system, TP, Ca, LDH, ALP, Mg or FRA is corrected based on the results of blank measurement performed in an alkaline blank measurement system, and T-Cho, TG, HDL-Cho or Alb is corrected based on the results of blank measurement performed in a surfactant blank measurement system.

The analyzing instrument of the present invention can be made to comprise a plurality of channels for moving a sample. In this case, an analysis reagent part or blank measurement reagent part is provided within each channel. The channels may be made to move sample by means of capillary action, or may be made to move sample using electrophoresis or the power of a micropump or pump. A plurality of channels are connected to one sample liquid inlet for example. Of course, it can also be constructed with a plurality of sample liquid inlets so that each channel is connected to a different sample liquid inlet.

The analyzing instrument of the present invention can be constructed so that a sample is directly applied to an analysis reagent part or blank measurement reagent part. In such an analyzing instrument, the analysis reagent parts and blank reagent parts are formed as absorbent carriers holding reagent or the like and fixed on a base.

The base used may be in the form of a sheet or film for example. Examples of materials for forming the base include polyethylene terephthalate, polyester, polypropylene, polyethylene, polyvinyl chloride, polyvinylidene chloride and polystyrene. A porous body in the form of a sheet or film for example can be used as the absorbent carrier. Examples of porous bodies include papers, foams, woven fabrics, nonwoven fabrics and knits. Examples of materials for forming the absorbent carrier include cotton, hemp, cellulose, nitrocellulose, cellulose acetate, rock wool, glass fiber, silica fiber, carbon fiber, boron fiber, polyamide, aramide, polyvinyl alcohol, polyvinyl acetate, rayon, polyester, nylon, polyacrylic acid, polyacrylic acid ester and polyolefin. There are no particular limitations on the form of these absorbent carriers, which are generally rectangular, long rectangular, circular or oval.

Of course, the analysis reagent part or blank reagent formed can be formed directly on the base without using an absorbent material.

### BRIEF DESCRIPTION OF THE DRAWINGS

Figure 1 is a total oblique view of an analyzing instrument according to the first embodiment of the present invention.
Figure 2 is a cross-section along line II-II in Figure 1.
Figure 3 is an exploded oblique view of the analyzing instrument shown in Figure 1.
Figure 4 is a table of analysis items which can be analyzed in the analyzing instrument shown in Figure 1, along with their abbreviations.
Figure 5 is a table of a plurality of blank measurement systems constructed in the analyzing instrument shown in Figure 1, along with the corresponding analysis items.
Figure 6 is a typical view showing the analyzing instrument of Figure 1 mounted on an analyzer.
Figure 7 is a total oblique view showing an analyzing instrument according to the second embodiment of the present invention.
Figure 8 is a total oblique view showing an analyzing instrument according to the third embodiment of the present invention.
Figure 9 is a total oblique view showing an analyzing instrument according to the fourth embodiment of the present invention.

### BEST MODE FOR CARRYING OUT THE INVENTION

The first through fourth embodiments of the present invention are explained in detail below with reference to the drawings.

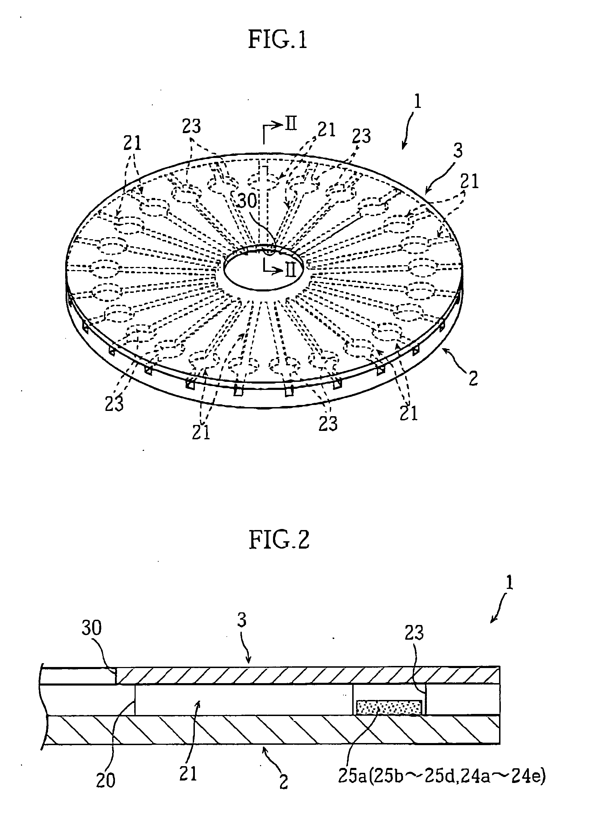

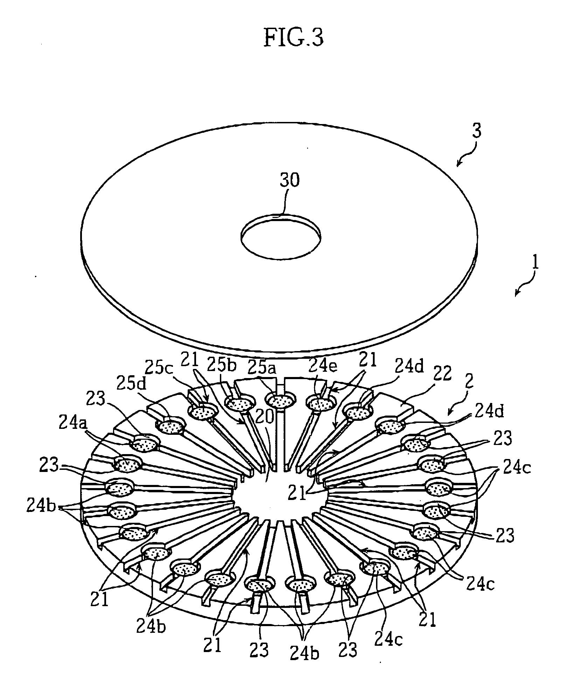

First, the first embodiment of the present invention is explained with reference to Figures 1 through 6.

The analyzing instrument 1 shown in Figures 1 through 3 of the present invention is constructed so as to move a sample using capillary action and analyze a sample by optical means.

Analyzing instrument 1 has substrate 2 and cover 3 laid over substrate 2. Substrate 2 is formed as a clear disk having liquid receiver 20 in the middle and multiple channels 21 extending radially from liquid receiver 20 towards the edge of substrate 2.

Liquid receiver 20 is for holding sample supplied to analyzing instrument 1 so that it can be introduced into channels 21. This liquid receiver 20 is formed as a circular indentation on upper surface 22 of substrate 2.

Channels 21 are for moving sample and are formed on upper surface 22 of substrate 2. Each channel 21 communicates with liquid receiver 20 and is open at the edge of analyzing instrument 1. Each channel 21 has a reaction site 23, and that part of each channel 21 excluding reaction site 23 has a roughly uniform rectangular cross-section. The width and depth dimensions of the rectangular section of each channel 21 are 10 to 500 µm and 5 to 500 µm for example, and are formed so that the width is half or more of the depth.

Reaction sites 23 have a cross-section greater than the main cross-section of channels 21. All reaction sites 23 are located on the same circle. Reaction sites 23 are provided with analysis reagent parts 24a through 24e and blank measurement reagent parts 25a through 25d.

Of analysis reagent parts 24a through 24e and blank measurement reagent parts 25a through 25d, analysis reagent parts 24a through 24e are for reacting and coloring specific components in the sample, and are in a solid form which dissolves when the sample is supplied. In this embodiment, 21 different analysis reagent parts 24a through 24e are prepared so that multiple analyses can be performed in analyzing instrument 1.

Analysis reagent parts 24a are for analyzing T-Bil or IP, and are made so as to construct an acidic reaction system (for example, pH 3.0 to 5.5) when a sample is supplied. Analysis reagent parts 24b are for analyzing Glu, UA, BUN, GOT, GPT, CPK, Amy, CGT or Cre, and are made so as to construct a neutral reaction system (for example, near pH 7.0) when a sample is supplied. Analysis reagent parts 24c are for analyzing TP, Ca, LDH, ALP, Mg or FRA, and are made so as to construct an alkaline reaction system (for example, pH 8.5 to 11.0) when a sample is supplied. Analysis reagent parts 24d and 24e are for analyzing T-Cho, HDL-Cho, TG or Alb, and comprise a surfactant. Analysis reagent parts 24d is made so as to construct a neutral reaction system (for example, near pH 7.0) when a sample is supplied. Analysis reagent part 24e is made so as to construct an acidic reaction system (for example, pH 3.0 to 5.5) when a sample is supplied.

Analysis reagent parts 24a through 24e are formed for example by applying a material liquid comprising a reagent (including surfactants) and a buffer to reaction site 23 and drying it. The reagents may be any capable of appropriately analyzing the aforementioned items, and known ones may be used. A nonionic surfactant isused as the surfactant so that lipids (such as triglyceride neutral fats) can be solubilized in the reaction system. A citric acid buffer is used as the buffer for analysis reagent parts 24a and 24e for example, a phosphoric acid buffer for analysis reagent parts 24b and 24d, and a CHES buffer, CAPS buffer of Good's buffer for analysis reagent parts 24c.

Blank measurement reagent parts 25a through 25d are used to correct for the effects of sample color and cloudiness (milkiness) caused by lipids. In this embodiment, 4 different blank measurement reagent parts 25a through 25d are prepared--acidic blank measurement reagent part 25a, neutral blank measurement reagent part 25b, alkaline blank measurement reagent part 25c and surfactant blank reagent part 25d. That is, in analyzing instrument 1 multiple analysis items share blank measurement reagent parts 25a through 25d in common, and 21 different analysis items can be managed by means of the four different blank measurement reagent parts 25a through 25d. Three different blank measurement reagent parts 25a through 25c with different pHs are prepared because the absorption wavelength peaks of bilirubin and hemoglobin, components which affect the color of the sample, are altered by the pH value of the reaction system, so their effect during analysis of a sample is dependent on pH. Moreover, surfactant blank measurement reagent part 25d is prepared because the degree of cloudiness (milkiness) of the sample system due to lipids differs depending on whether or not a surfactant which solubilizes lipids is present.

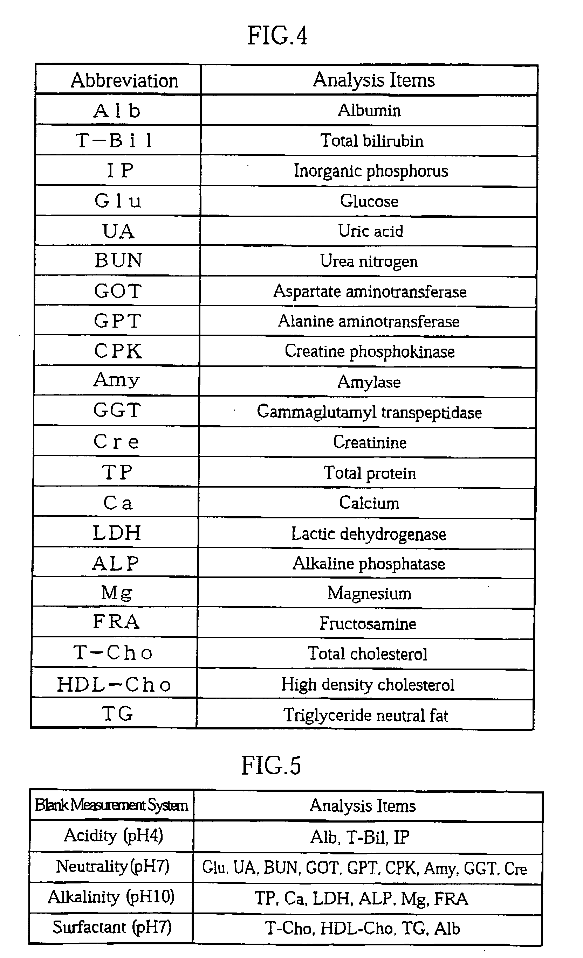

As shown in Figure 5, acidic blank measurement reagent part 25a is for blank measurement of albumin (Alb), total bilirubin (T-Bil) and inorganic phosphorus (IP).

Neutral blank measurement reagent part 25b is for blank measurement of glucose (Glu), uric acid (UA), urea nitrogen (BUN), aspartate aminotransferase (GOT), alanine aminotransferase (GPT), creatine phosphokinase (CPK), amylase (Amy), gammaglutamyl transpeptidase (GGT) and creatinine (Cre).

Alkaline blank measurement reagent part 25c is for blank measurement of total protein (TP), calcium (Ca), lactic dehydrogenase (LDH), alkaline phosphatase (ALP), magnesium (Mg) and fructosamine (FRA).

Surfactant blank measurement reagent part 25d is for blank measurement of total cholesterol (T-Cho), high density cholesterol (HDL-Cho) and triglyceride neutral fat (TG).

Blank measurement reagent parts 25a through 25c are formed by applying a buffer to reaction part 23 and drying it. Blank measurement reagent part 25d is formed by applying a material liquid comprising a buffer and a surfactant for example to reaction part 23 and drying it. A malic acid buffer or other carboxylic acid buffer for example is used as the buffer for blank measurement reagent part 25a, a phosphoric acid buffer for example for blank measurement reagent parts 25b and 25d, and a carbonic acid buffer, glycine buffer or Good's buffer for blank measurement reagent part 25c. A nonionic surfactant is used as the surfactant as in the case of analysis reagent parts 24d and 24e.

As shown in Figures 1 through 3, substrate 2 of analyzing instrument 1 is formed by resin molding using a clear resin material such as poly-methyl methacrylate (PMMA) or another acrylic resin or polystyrene (PS), polycarbonate (PC), or polyethylene terephthalate (PET). Liquid receiver 20 and multiple channels 21 are incorporated during the aforementioned resin molding by manipulating the shape of the mold.

The inner surfaces of liquid receiver 20 and multiple channels 21 are preferably given a hydrophilic treatment. Various known methods can be adopted for hydrophilic treatment, which is however preferably performed by first bringing a mixed gas comprising fluorine gas and oxygen gas for example into contact with each inner surface and then bringing water or steam into contact with each inner surface. In this method, because hydrophilic treatment is accomplished using gas and water it can be reliably applied even to standing surfaces (the sides of channels and the like) which are hard to treat by known hydrophilic treatment methods using ultraviolet irradiation. Hydrophilic treatment of each surface is performed so that the contact angle with respect to water is 0 to 80 degrees or preferably 0 to 60 degrees.

Cover 3 is formed as a clear disk having sample inlet 30. Sample inlet 30 is used when introducing sample liquid, and is formed as a through hole. Sample inlet 30 is formed in the center of cover 3 so as to be directly over liquid receiver 20 of substrate 2.

This cover 3 can be formed by drawing or resin molding using a clear resin material as for substrate 2. Liquid receiver 20 can be formed by punching when cover 3 is formed by drawing, and can be incorporated during resin molding when cover 3 is formed by resin molding. Hydrophilic treatment is also preferably applied to at least those parts of cover 3 which face channels 21 of substrate 2. The methods adopted for hydrophilic treatment can be similar to those for hydrophilic treatment of substrate 2.

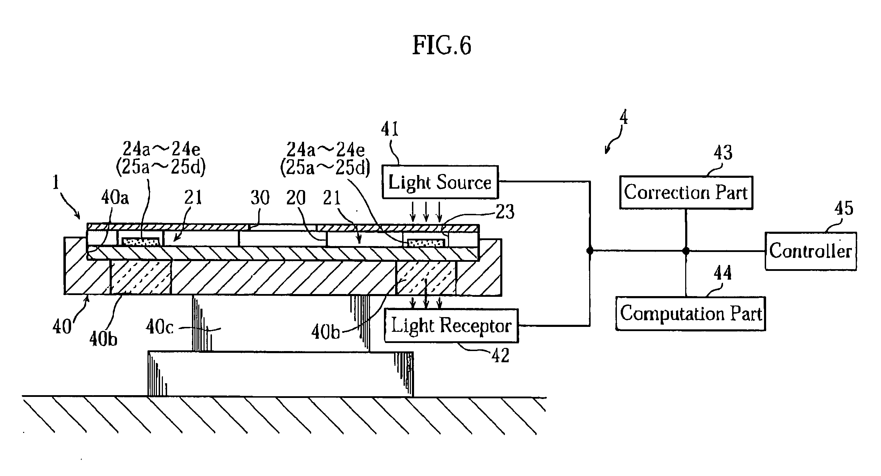

The analyzing instrument 1 explained above is used mounted on the analyzer 4 shown in Figure 6 for example. Analyzer 4 is equipped with mount 40, light source 41, light receptor 42, correction part 43, computation part 44 and controller 45.

Mount 40 is provided with indentation 40a for holding analyzing instrument 1 and light-transmitting region 40b. This mount 40 is supported by rotating shaft 40c, and is configured so that mount 40 is rotated by the rotation of rotating shaft 40c. Rotating shaft 40c is connected to a drive mechanism (not shown), and is controlled so as to rotate at angles corresponding to the arranged pitch of reaction sites 23 on analyzing instrument 1. Light-transmitting region 40b is provided in a location corresponding to reaction sites 23 of analyzing instrument 1 when analyzing instrument 1 is mounted in indentation 40a. This light-transmitting region 40b is formed by comprising the target site of mount 40 of a clear resin or other clear material. Of course, all of mount 40 can also be formed of clear material.

Light source 41 is for illuminating reaction sites 23 of analyzing instrument 1. Light source 41 is comprised for example by a mercury lamp or white LED. When these light sources are used the light from light source 41 is passed through a filter before illuminating reaction sites 23, although this is not shown in the figures. This is because light of a wavelength matched to the light absorption characteristics of a component to be analyzed in the reaction liquid is selected by the filter. The light source can also be composed of multiple light sources with different peak wavelengths.

Light receptor 42 is for receiving light passing through reaction sites 23, and is positioned on the same axis as light source 41 on the other side of light-transmitting region 40b. The amount of light received by this light receptor 42 is the basis for analyzing the sample (computing concentrations and determining correction values for example). Light receptor 42 is comprised for example by a photodiode.

Correction part 43 is for computing correction values based on results for light received by light receptor 42 when reaction sites 23 having blank measurement reagent parts 25a through 25d have been illuminated with light from light source 41. Computation part 44 is for performing computations for sample analysis based on the received light results from light receptor 42 and the correction values computed by correction part 43 when reaction sites 23 having analysis reagent parts 24a through 24e have been illuminated with light from light source 41. Controller 45 is for controlling the operations of parts 41 through 44.

Correction part 43, computation part 44 and controller 45 are constructed by connecting multiple memories (such as ROM or RAM) to one CPU.

During sample analysis, analyzing instrument 1 is mounted on mount 40 of analyzer 4 as shown in Figure 6. Sample is supplied to liquid receiver 20 of analyzing instrument 1 via sample inlet 30. Sample supplied to liquid receiver 20 moves by capillary action through the channels towards the edge of analyzing instrument 1. In this way, sample is supplied all at once through channels 21 to multiple reaction sites 23.

In each reaction site 23, analysis reagent parts 24a through 24e or blank measurement parts 25a through 25d are dissolved by the sample. In this way, liquid phase reaction systems are constructed in reaction sites 23 having analysis reagent parts 24a through 24e. The sample and reagent react in the liquid phase reaction system, and for example the liquid phase reaction system exhibits color correlated to the amount of the component to be detected in the sample, or else a reaction product is produced corresponding to the amount of the component to be detected. As a result, the liquid phase reaction systems constructed in reaction sites 23 exhibit translucence (light absorbency) corresponding to the amount of the component to be detected.

Meanwhile, blank measurement systems are constructed in reaction sites 23 having blank measurement reagent parts 25a through 25d. More specifically, an acidic blank measurement system with a pH of 4.0 for example in constructed in a reaction site 23 having blank measurement reagent part 25a, a neutral blank measurement system with a pH of 7.0 for example is constructed in a reaction site 23 having blank measurement reagent part 25b, an alkaline blank measurement system with a pH of 10.0 for example is constructed in a reaction site 23 having blank measurement reagent part 25c, and a surfactant blank measurement systems having a pH of 7.0 for example and comprising a surfactant is constructed in a reaction site 23 having blank measurement reagent part 25d.

When a specific time has passed since sample was supplied to reaction sites 23, reaction sites 23 are illuminated with light from light source 41, and the amount of transmitted light is measured at light receptor 42. Illumination with light from light source 41 and reception of transmitted light by light receptor 42 are performed for all reaction sites 23 set on all channels 21 as mount 40 is rotated at fixed angles. At this time, correction values are computed at correction part 43 based on amount of light transmitted at reaction sites 23 having blank measurement reagent parts 25a through 25d. More specifically, in correction part 43 correction values are computed for T-Bil and IP based on amount of transmitted light from reaction site 23 having blank measurement reagent part 25a, correction values are computed for Glu, UA, BUN, GOT, GPT, CPK, Amy, CGT and Cre based on amount of transmitted light from reaction site 23 having blank measurement reagent part 25b, correction values are computed for TP, Ca, LDH, ALP, Mg and FRA based on amount of transmitted light from reaction site 23 having blank measurement reagent part 25c, correction values are computed for T-Cho, TG and HDL-Cho based on amount of transmitted light from reaction site 23 having blank measurement reagent site 25d, and correction values are computed for Alb based on amount of transmitted light from reaction site 23 having blank measurement reagent part 25a and reaction site 23 having blank measurement reaction part 25d.

Sample analysis such as for example computing the concentration of a component to be detected or confirming the present or absence of a component to be detected is performed in computation part 44 based on the computed results of correction data from correction part 43 and the amount of transmitted light in reaction parts 23 having analysis reagent parts 24a through 24e,. More specifically, for example the amount of light received by light receptor 42 (or the absorbance derived from this) is multiplied by a correction value, and the concentration of the component to be detected is computed by applying the corrected value to a previously prepared working curve, or else the presence or absence of a component to be detected is confirmed by determining whether the corrected value is greater than a previously determined threshold.

In the correction method of this embodiment, multiple analysis items are grouped into four groups according to type of reaction system (pH and presence or absence of surfactant in this embodiment), with a blank measurement being common to all analysis items constituting a group. Consequently, the number of blank measurements need only be the number of analysis item groups and not the number of individual measurement items as in the past. For example, in this embodiment there are 21 analysis items but only four blank measurements. As a result, the amount of reagent and amount of sample required for blank correction and for analysis as a whole is reduced to the extent that the number of blank measurements is reduced. In this way, the costs of the analyzing instrument are reduced, and when the sample is a biological sample such as urine or blood the burden on the test subject when the sample is obtained is reduced. When the number of blank measurements is small fewer regions for blank measurement need to be included in analyzing instrument 1, so analyzing instrument 1 can be made smaller.

The analyzing instrument 1 of this embodiment is constructed so as to analyze a sample using transmitted light, but either substrate 2 or cover 3 could also be made opaque, and the sample analyzed using regular reflected light or scattered light. Moreover, the arrangement of multiple channels 21 does not necessarily have to be radial.

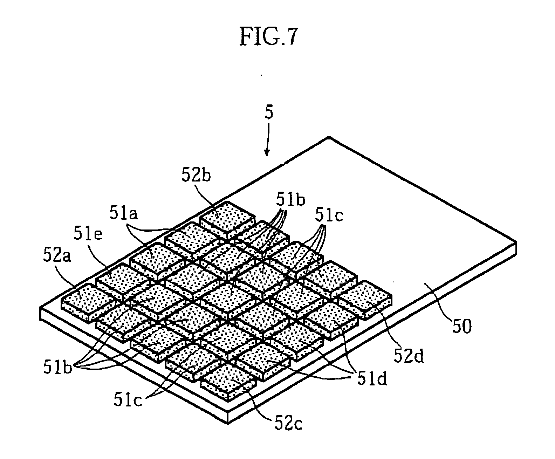

Next, an analyzing instrument according to the second embodiment of the present invention is explained with reference to Figure 7.

The analyzing instrument 5 shown in Figure 7 has multiple analysis reagent pads 51a through 51e and multiple blank measurement pads 52a through 52d arranged in a matrix on a base 50. Analyzing instrument 5 can be constructed so that base 50 is transparent and samples are analyzed based on transmitted light, or so that base 50 is opaque and samples are analyzed based on regular reflected light or scattered light.

Analysis reagent pads 51a through 51e each comprise a reagent matched to an analysis item for example. Analysis reagent pads 51a relate to analysis items (other than Alb) which are measured in an acidic reaction system. Analysis reagent pads 51b relate to analysis items which are measured in a neutral reaction system. Analysis reagent pads 51c relate to analysis items which are measured in an alkaline reaction system. Analysis reagent pads 51d relate to analysis items (other than A1b) which are measured in a surfactant reaction system. Analysis reagent pad 51e relates to A1b (see Figure 5).

Likewise, blank measurement pads 52a through 52d are of four types (see Figure 5)--acidic blank measurement pad 52a capable of constructing an acidic blank measurement system, neutral blank measurement pad 52b capable of constructing a neutral blank measurement system, alkaline blank measurement pad 52c capable of constructing an alkaline blank measurement system, and surfactant blank measurement pad 52d, containing a surfactant and capable of constructing a neutral blank measurement system.

In analyzing instrument 5 as well multiple analysis items are divided into four groups according to the attributes and composition of the reaction system, so that a blank measurement can be shared by all analysis items in a group. Consequently, the effects of analyzing instrument 1 (see Figures 1 through 3) explained in the first embodiment of the present invention can be obtained with analyzing instrument 5.

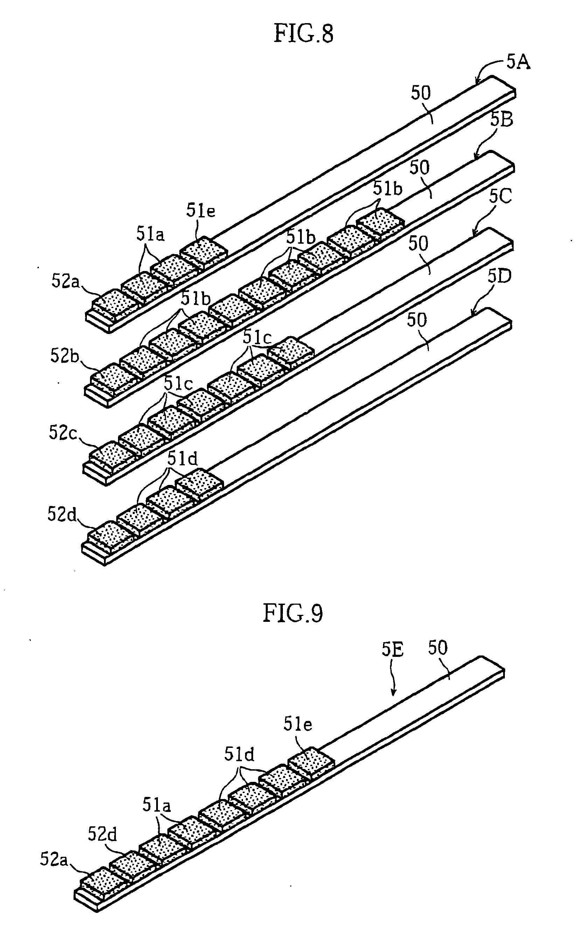

Next, an analyzing instrument of the third embodiment of the present invention is explained with reference to Figure 8.

In Figure 8 the elements having the same codes as in Figure 7 are the same as in Figure 7. The analyzing instrument 5A (5B-5D) shown in Figure 8 has multiple analysis reagent pads 51a (51b-51e) and one blank measurement pad 52a (52b-52d) fixed on base 50. Multiple analysis reagent pads 51a (51b-51e) corresponding to analysis items with similar reaction systems and one blank measurement pad 52a (52b-52d) corresponding to these analysis items are included in each analyzing instrument 5A (5B-5D).

Analyzing instruments 5A through 5D can be used individually or the four analyzing instruments 5A through 5D can be used as a set. In either case, because analyzing instruments 5A through 5D are also configured with blank measurements shared according to the attributes and composition of the reaction system, the effects of analyzing instrument 1 (see Figures 1 through 3) explained in the first embodiment of the present invention can be obtained.

Next, an analyzing instrument according to the fourth embodiment of the present invention is explained with reference to Figure 9. In Figure 9 elements marked with the same codes as in Figure 7 are the same as in Figure 7.

The analyzing instrument 5E shown in Figure 9 has multiple analysis reagent pads 51a, 51d and 51e and two blank measurement pads 52a and 52d fixed on base 50. That is, the construction of analyzing instrument 5E is a combination of analyzing instruments 5A and 5D shown in Figure 8, with analysis items of three types of reaction systems, an acidic system, a surfactant system and an acidic surfactant system, being included in analyzing instrument 5E. Correspondingly, analyzing instrument 5E is provided with acidic blank measurement pad 52a and surfactant blank measurement pad 52d.

In the embodiments described above reaction systems were classified according to the conditions of reaction system pH (acidic, neutral or alkaline) and presence or absence of a surfactant as an example, but the classification of reaction systems is not limited to that described above, and another classification is possible. Moreover, the analyzing instrument 1 of the form explained with reference to Figures 1 through 3 may also be configured with one or two blank measurement systems as in analyzing instruments 5A-5D and 5E in Figures 8 and 9.

## Claims

1. A method of correction during sample analysis in a method of performing analysis with respect to a plurality of analysis items on the basis of a reaction liquid from reaction of a sample and a reagent, a method applying correction when analyzing said analysis items, wherein correction is applied based on the same blank measurement results with respect to those plurality of analysis items out of said plurality of analysis items for which the reaction conditions during analysis are similar.

2. A method of correction during sample analysis according to Claim 1, wherein said plurality of analysis items are separated into a plurality of groups with the members of each group sharing similar reaction conditions during analysis, a plurality of blank measurements with measurement conditions different from one another are performed corresponding to said plurality of groups, and correction is applied based on the blank measurement results corresponding for each of the groups in analyzing the individual analysis items that make up the group.

3. A method of correction during sample analysis according to Claim 1, wherein correction is performed based on the results of two or more blank measurements when analyzing some of said plurality of types of analysis items.

4. A method of correction during sample analysis according to Claim 1, wherein, said reaction conditions are the pH of said reaction liquid.

5. A method of correction during sample analysis according to Claim 1, wherein said reaction conditions are whether or not a surfactant is included as said reagent in said reaction liquid.

6. A method of correction during sample analysis according to Claim 1, wherein said blank measurement is performed in at least one measurement system selected from an acidic blank measurement system, a neutral blank measurement system, an alkaline blank measurement system and a surfactant blank measurement system comprising a surfactant.

7. A method of correction during sample analysis according to Claim 6, wherein said surfactant blank measurement system is adjusted to a neutral pH range.

8. A method of correction during sample analysis according to Claim 7, wherein in analyzing some of said plurality of types of analysis items correction is performed based on both the results of blank measurement in said surfactant blank measurement system and the results of blank measurement in said acidic blank measurement system or said alkaline blank measurement system.

9. A method of correction during sample analysis according to Claim 1, wherein said sample is urine or blood.

10. A method of correction during sample analysis according to Claim 9, wherein said plurality of analysis items comprise at least one item selected from the group consisting of albumin (Alb), total bilirubin (T-Bil), inorganic phosphorus (IP), glucose (Glu), uric acid (UA), urea nitrogen (BUN), aspartate aminotransferase (GOT), alanine aminotransferase (GPT), creatine phosphokinase (CPK), amylase (Amy), gammaglutamyl transpeptidase (GGT), creatinine (Cre), total protein (TP), calcium (Ca), lactic dehydrogenase (LDH), alkaline phosphatase (ALP), magnesium (Mg), fructosamine (FRA), total cholesterol (T-Cho), high density cholesterol (HDL-Cho) and triglyceride neutral fat (TG).

11. An analyzer for analyzing a plurality of specific components in a sample on the basis of a reaction liquid from reaction of a sample and a reagent, comprising computation means for performing computations necessary for analyzing a plurality of specific components in a sample, wherein, said computation means is constructed so as to apply correction based on correction data obtained based on the same blank measurement results for a plurality of specific components having similar reaction conditions during analysis when performing computations for analyzing said plurality of specific components.

12. An analyzer according to Claim 11, wherein said computation means is constructed so as to perform correction based on the results of two or more blank measurements in analysis of some of said plurality of specific components.

13. An analyzer according to Claim 11, further comprising correction means for obtaining said correction data based on blank measurement results.

14. An analyzing instrument comprising a plurality of analysis reagent parts each comprising a different reagent and one or more blank measurement reagent parts, wherein said one or plurality of blank reagent parts are shared by those of said plurality of analysis reagent parts which have similar reaction conditions during analysis.

15. An analyzing instrument according to Claim 14, wherein said one or plurality of blank measurement reagent parts include at least one selected from an acidic blank measurement reagent part, a neutral blank measurement reagent part, an alkaline blank measurement reagent part and a surfactant blank measurement reagent part comprising a surfactant.

16. An analyzing instrument according to Claim 14 comprising a plurality of channels for moving a sample, wherein said analysis reagent parts or said blank measurement reagent parts are provided within said channels.

17. An analyzing instrument according to Claim 16, wherein said plurality of channels are constructed so as to move a sample by capillary action.

18. An analyzing instrument according to Claim 16, wherein said plurality of channels are connected to one sample inlet.

19. An analyzing instrument according to Claim 14, wherein said plurality of analysis reagent parts and said one or plurality of blank measurement reagent parts are constructed so that sample is applied directly.

20. An analyzing instrument according to Claim 19, wherein said analysis reagent parts and said blank measurement reagent parts have a construction in which reagent is held on an absorbent carrier fixed on a base.
